# EUROPEAN PATENT SPECIFICATION

(11) **EP 3 222 319 B1**
(45) Date of publication and mention of the grant of the patent: **12.06.2019**
(21) Application number: 16162466.3
(22) Date of filing: 25.03.2016
(51) Int. Cl.: A61N 1/39, A61N 1/362, A61N 1/368

(54) **HEART STIMULATION DEVICE**
HERZSTIMULATIONSVORRICHTUNG
DISPOSITIF DE STIMULATION CARDIAQUE

(43) Date of publication of application: 27.09.2017
(73) Proprietor: P.A. & M. PARTECIPAZIONI AZIONARIE & MANAGEMENT S.R.L., 00199 Roma (IT)
(72) Inventor: DE BELLIS, Ferruccio, 00198 Roma (IT)
(74) Representative: Celona, Antonio

(56) References cited:
- EP-A1- 0 562 237
- EP-A1- 2 881 140
- WO-A1-2013/021301
- US-A- 5 267 560
- US-A- 5 395 397
- US-A1- 2008 109 043
- US-A1- 2012 239 102
- US-B2- 7 181 284

## Description

### Field of the invention

The present invention relates to a dual chamber pacemaker or defibrillator.

### Background of the invention

The cardiac muscle is an electro-mechanical pump with four chambers: Right Atrium RA, Left Atrium LA, Right Ventricle RV and Left Ventricle LV. Clinical conditions often require the implantation of a pacemaker (PM) or defibrillator (Def). The first pacemakers (PMs) were provided with a single electrode, which was implanted on one or two ventricles of the heart of the sick patient, and epicardial stimulation was only performed on the ventricle to which said electrode was connected in those pathological cases in which the heart did not contract on its own or did not adequately contract. The implants were performed with a thoracotomy or subxiphoid approach. In 1967, pacemaker implantation became endocardial, i.e. the stimulating electrode was inserted transvenously into the right ventricle; the intervention is considerably less invasive.

In a subsequent development phase, greater clinical knowledge, improved implantation techniques, significant evolution of the pacemakers both from a stoichiometric and circuital point of view, and significant improvement of the stimulating electrodes, it was also proposed to place only one electrode in the atrium, as an alternative to connecting the electrode to the ventricle alone, a much more physiological solution in the case of particular clinical pictures.

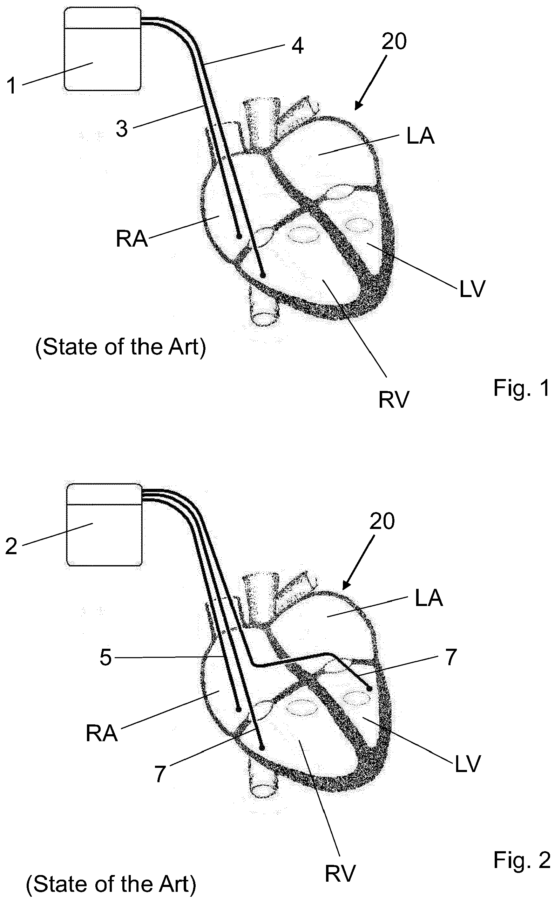

In a subsequent phase pacemakers 1 (Fig. 1), which had two electrodes 3 and 4, were also developed, and in this case an electrode 4 was positioned in the right ventricle and the other electrode 3 was positioned in the right atrium. The surgical operation for implantation of these pacemakers, known as dual-chamber or sequential pacemakers, was more complicated given the presence of two electrodes, thus they were only preferred in those heart conditions that justified the increased risk. Subsequently, with the progress of implantation techniques and the progress of the electrode technology, there was an increasing choice of dual-chamber pacemakers; since 1995, implanting a pacemaker means implanting a dual-chamber pacemaker unless there are clinical contraindications.

In patients with an implanted pacemaker (PM) or defibrillator (Def), the modalities of electric stimulation of the cardiac chambers have a determinant effect on the blood pumping capabilities. In nature the LV contracts a few milliseconds (msec) before the RV; if the VD contracts before the LV, this event is considered an anomaly called Left Branch Block (LBB). As mentioned, patients carrying a PM or Def have two implanted electrodes, one in the RA and the second in the RV. In order to induce the cardiac contraction, the PM, or Def, delivers an electric pulse in the RA and a second electric pulse in the RV.

Obviously, by stimulating only the RV, the LV will contract later, thus inducing an LBB more or less severe according to the delay of the contraction of the LV; the longer the delay, the more important is the LBB.

The LBB, either natural or induced by electric stimulation of the RV, is one of the main causes of Heart Failure (HF). Heart Failure is a disease suffered by over one million Italians over 50 years of age. It occurs when the heart is unable to pump blood in quantity necessary to supply oxygen to meet the body's needs. Therefore, the implant of a PM, or Def, induces the LBB, thus posing the basis for Herat Failure.Given their clinical conditions, some patients require particular, more complex stimulations than the sequential, right atrium-ventricle stimulation. Such patients have serious conditions, i.e. they have an ejection fraction of less than 35% and are in NYHA class III or IV. In order to improve the clinical picture, i.e. to improve stroke volume and cardiac output, in addition to the atrial electrode 5 (Fig. 2) in the right atrium RA and the right ventricular electrode 6 in the right ventricle RV, in these patients there is also inserted a third electrode 7 in the left ventricle LV to also stimulate this part of the heart 20 with programmed times compared to the stimulation of the right ventricle. In such patients, depending on the clinical picture, there can be implanted a defibrillator 2 instead of a pacemaker. It should be borne in mind that a defibrillator also performs the functions of a pacemaker and that such functions are suitably controlled by the control programme of the pacemaker-defibrillator device.

For these patients, for whom it is necessary to implant a third electrode in the left ventricle in addition to the two electrodes in the right ventricle and atrium, there is nevertheless envisaged an implantation surgical operation that is often particularly lengthy and therefore very dangerous due to the fact that it is performed on patients whose heart is particularly weakened, with risk to their life in the course of the operation. The difficulty of such an operation results from the need to make the third electrode cross the coronary sinus to reach the affixing position above the left ventricle inside the great cardiac vein. There are sometimes particular difficulties that advise against implanting the third electrode adapted to stimulate the left ventricle, and that are generally linked to the clinical conditions of the patient. WO2013021031 discloses a pacemaker with the features of the preamble of claim 1.

### Summary of the invention

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

An object of the present invention is to provide a dual-chamber pacemaker or defibrillator with improved stimulation sequence, which allows the achievement, with just two electrodes, of the same or improved haemodynamic advantages obtained with a triple-chamber implant having three electrodes, thus achieving a therapeutic effect.

Another object of the present invention is to provide a dual-chamber pacemaker or defibrillator, which prevents the Left Branch Block and, therefore, Heart Failure. These and other objects that will be apparent in light of the following description, are achieved by a heart stimulation device to stimulate the heart of a patient with a sequence of cardiac stimulation cycles, the heart stimulation device comprising, according to claim 1,
- an atrial electrode adapted to be connected to the right atrium RA of the heart,
- a ventricular electrode adapted to be connected to the right ventricle RV of the heart, and
- a control system programmed to emit, for each cardiac stimulation cycle:
   - an atrial impulse for a stimulation of the right atrium by means of the atrial electrode,
   - a first ventricular impulse in the right ventricle RV by means of the ventricular electrode, emitted after a time interval T1 from the emission of the atrial impulse,
- a second ventricular impulse for a stimulation of the right ventricle RV, emitted after a time interval T2 from the first ventricular impulse, having a Voltage and a duration adapted to provoke the cardiac stimulation of the patient,
wherein the first ventricular impulse is formed by
a first part having a constant Voltage which is not in excess of 50% of the cardiac stimulation threshold potential of the patient, and
a second part, subsequent to the first part, having an absolute value of Voltage which is increasing from said constant Voltage up to 70-90% of the cardiac stimulation threshold potential of the patient,
the first part and the second part having equal duration, and
the absolute value of voltage increasing linearly.

The heart stimulation device of the invention is for patients with particular heart conditions which necessarily require the implantation, or with indication for implantation, of a pacemaker or defibrillator. In particular, the heart stimulation device of the invention is intended for those patient with a severe heart condition which requires the implantation of a three-chambers pacemaker or defibrillator.

In this case, the heart stimulation device of the invention has therapeutic effects, mainly due to its capability of considerably increasing the stroke volume and cardiac output. Indeed, the stroke volume and cardiac output achieved with a heart stimulation device of the invention are comparable or increased with respect to a pacemaker, or defibrillator, of the three-chambers type.

Also, the heart stimulation device of the invention is for patients which necessarily require the implantation, or with indication for implantation, of a pacemaker or defibrillator, in particular of the dual chamber type. Indeed, the stimulation of the heart stimulation device of the invention, also called FDB stimulation, eliminates the negative haemodynamic effect of LBB and, as mentioned, considerably increases both the stroke volume and the cardiac output.

In addition, thanks to the invention, the important advantage of reducing the invasiveness of the implant operation of the device is achieved.

According to the invention, the first ventricular impulse, defined below-threshold impulse or below threshold stimulus, is below the threshold voltage, preferably below the set of threshold values of voltage and duration, to provoke a cardiac stimulation. The first ventricular impulse does not induce the cardiac contraction. Such first ventricular impulse has a constant voltage part followed by an increasing voltage part.

Advantageously, the inventor has found that such specific first ventricular impulse causes a pre-excitation that contributes to more quickly transmitting the second ventricular impulse, thus significantly improving the stroke volume and cardiac output. Due to the first ventricular impulse, the second ventricular impulse not only propagates much faster, but also in a more physiological way as compared with the electrical stimulation of the right ventricle with only one impulse. Also, the first ventricular impulse according to the invention provides a stroke volume and cardiac output 15-20% higher with respect to other forms of pre-stimuli.

The dependent claims describe preferred embodiments of the invention.

### Brief description of the drawings

Further characteristics and advantages of the invention will become clearer in light of the detailed description of a preferred but non-exclusive embodiment of a stimulation device, and of a control process thereof, for the stimulation produced by a pacemaker or defibrillator, illustrated by way of a non-limiting example, with the aid of the accompanying drawings, wherein:
Figure 1 shows a schematic, cross-sectional view of a heart with sequential, dual-chamber stimulation device with two electrodes implanted as in the prior art;
Figure 2 shows a schematic, cross-sectional view of a heart with sequential, three-chamber stimulation device with three electrodes implanted as in the prior art;
Figure 3 shows a schematic, cross-sectional view of a heart associated with a, dual-chamber sequential stimulation device, with two electrodes implanted and programmed according to the invention.
Figure 4 shows a diagram of the stimulation of the human heart by means of a pacemaker or defibrillator programmed according to the invention,
Figure 5 shows an enlarged detail of Figure 4.

The same reference numbers in the drawings identify the same elements or components.

### Detailed description of preferred embodiments of the invention

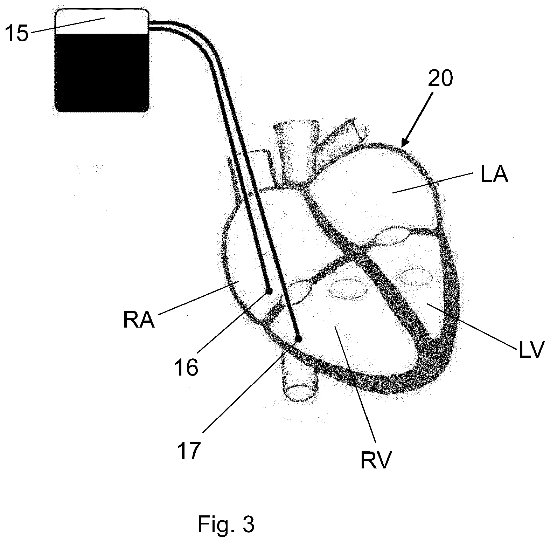

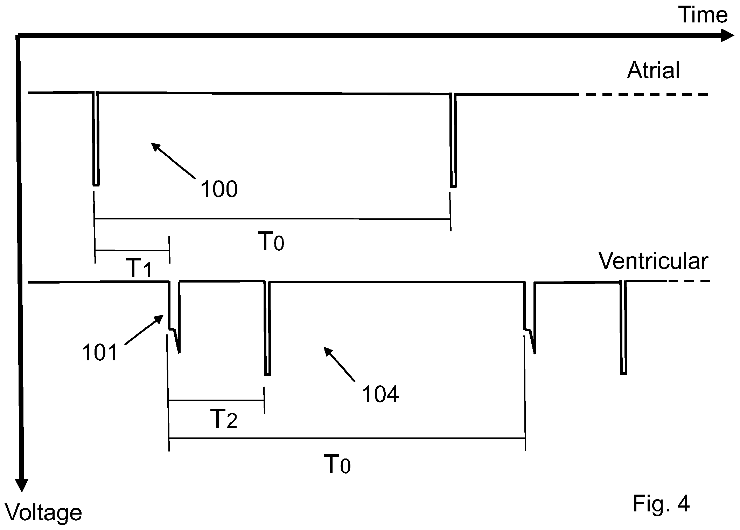

With reference to Figures 3-5, the invention provides a heart stimulation device 15, in particular a pacemaker or defibrillator. In particular, it is a heart stimulation device 15 of the dual-chamber type, preferably designed for a patient having an ejection fraction less than 35% and classified in NYHA class III or IV.

The heart stimulation device 15 has two electrodes 16, 17. In particular, An atrial electrode 16 configured to be connected to the right atrium RA, and a ventricular electrode 17 configured to be connected to the right ventricle RV. The stimulation device 15 is programmed, or configured, to allow the ventricular electrode 17 to emit two stimuli in the course of the same cardiac stimulation cycle. Suitable means, such as an electronic control system, known to those skilled in the art, can be programmed in order to achieve this result.

The electrodes 16, 17 are respectively positioned inside the right atrium RA and the right ventricle RV on the basis of the intraoperative measures, such as in the implant, normally without other precautions, while exclusively following the usual rules known to all expert implanters of this type of implant.

The natural contraction between the left ventricle LV and the right ventricle RV in human hearts occurs at a time interval comprised between 5 and 20 msec. Should this time interval exceed 50 msec, the heart pumps incorrectly.

The succession of the two stimuli in the right ventricle RV must be made in a very particular way to permit correct contraction of the left ventricle LV so that there is good stroke volume and cardiac output. This determines the programming of the pacemaker when it is implanted in the patient's body.

Thus in accordance with the invention, the control system is programmed to emit, for each cardiac stimulation cycle:
- an atrial impulse 100 for the stimulation of the right atrium RA by means of the atrial electrode 16,
- a first ventricular impulse 101 in the right ventricle RV by means of the ventricular electrode 17, emitted after a time interval T1 from the emission of the atrial impulse 100,
- a second ventricular impulse 104 for a stimulation of the right ventricle RV, emitted after a time interval T2 from the first ventricular impulse 101, having a Voltage and a duration adapted to provoke the cardiac stimulation of the patient. The first ventricular impulse 101 is formed by
a first part 102 having a constant Voltage which is not in excess of 50% of the cardiac stimulation threshold potential of the patient, and
a second part 103, subsequent to the first part 102, having an absolute value of Voltage which is increasing from said constant Voltage up to 70-90%, preferably up to 80-90%, of the cardiac stimulation threshold potential of the patient.

In particular, the absolute value of voltage of the second part 102 of the first ventricular impulse 101 increases up to a maximum value of voltage having a value up to 70-90%, preferably up to 80-90%, of the cardiac stimulation threshold potential of the patient.

Preferably, such absolute value of voltage increases linearly, or according to a monotonically increasing function, during the second part 104 of the first ventricular impulse 101.

The first part 102 has preferably a duration of about 0,4 msec, or about 0,5 msec; more preferably the duration is of 0,40 msec. The second part 103 has a duration has preferably a duration of about 0,4 msec, or about 0,5 msec; more preferably the duration is of 0,40 msec. The first part 102 has a constant voltage which is preferably 35-50%, more preferably 50%, of the cardiac stimulation threshold potential of the patient.

According to the embodiment shown in figures, the first part 102 and the second part 103 have equal duration.

The time interval T1 between the emission of the atrial impulse 100 and the emission of the first ventricular impulse 102 is preferably comprised between about 60 and about 120 msec, for example about 80 msec.

Generally, The time interval T₂ between the first ventricular impulse 101 and the second ventricular impulse 104 is identified by detection methods of the known type by means of the programmer of the implanted pacemaker or defibrillator.

Preferably, the time interval T2 between the emission of the first ventricular impulse 102 and the emission of the second ventricular impulse 104 is 70 msec. This value allows to achieve best results.

The time interval T1+T2 is defined as atrioventricular delay AV, while T2 is defined ventricular sequence delay.

The atrial impulse 100 marks the start of cardiac stimulation cycle, which has an overall duration T₀. In particular, the time interval T₀ is between the emission of the atrial impulse 100 of a cardiac stimulation cycle and the atrial impulse of the subsequent cardiac stimulation cycle. Also, it is preferred that the same time interval T₀ is between the emission of the first ventricular impulse 101 of a cardiac stimulation cycle and the first ventricular impulse of the subsequent cardiac stimulation cycle. Preferably, T₀ is comprised between about 750 and about 1000 msec.

This first ventricular impulse 101 is defined "below threshold stimulus" or pre-stimulus" or "below-threshold impulse" for the purposes of this description. It is produced with an impulse performed "BELOW THRESHOLD". In particular, it is performed below the Voltage, preferably below the set of Voltage and duration, required to provoke a cardiac stimulation. In other words, the first ventricular impulse 101 does not provoke a cardiac stimulation, or contraction, but pre-excites the ventricular endocardium, preferably only the ventricular endocardium.

Typically, the cardiac stimulation threshold potential of the patient is comprised between 0,80 V and 1,40 V, and the threshold duration is between about 0,4 and 0,5 msec.

By way of non-limiting example, if for a certain patient the stimulation of the heart 20 requires the amplitude of 1 Volt (threshold value) for an impulse duration equal to 0,50 msec at such Voltage, the second part 103 of the first ventricular impulse 101 has a maximum absolute value of Voltage equal to or lower than 70 to 90%, preferably 80-90% of the threshold potential. According to the invention, the first ventricular impulse does not cause the same contractive effects on the cardiac muscles as a normal atrial or ventricular stimulus, in particular does not cause a cardiac contraction.

By contrast, the second ventricular impulse 104 is adapted to provoke a cardiac contraction of an amplitude (i.e. Voltage value) and duration equal to, or above the threshold values, and that is preferably in line with the Safety Margin rules.

Preferably, the second ventricular impulse has a duration of about 0,4 or about 0,5 msec. Also, it is preferred that the atrial impulse has a duration of about 0,4 or about 0,5 msec.

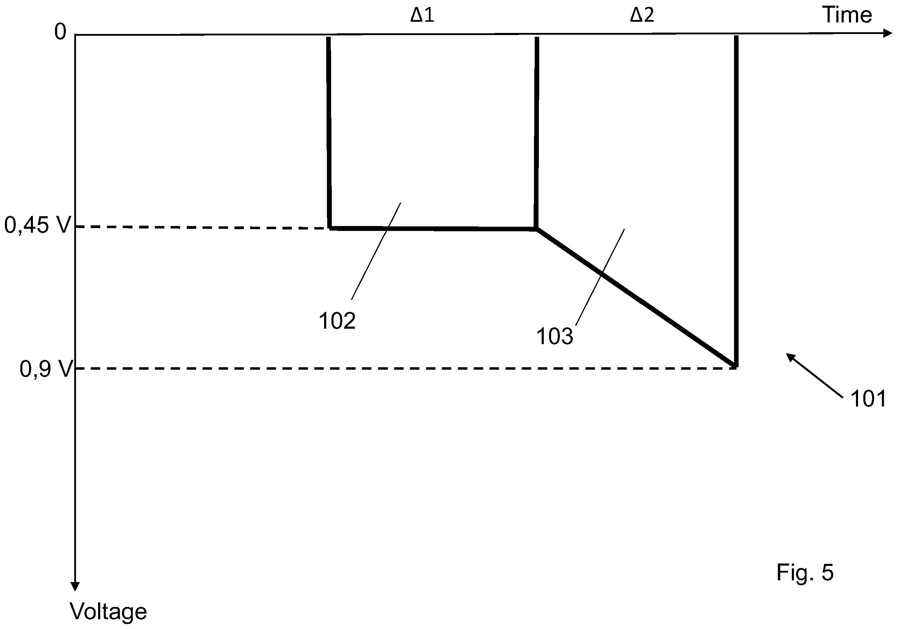

As shown in Figure 5, the first part 102 has a constant Voltage of 0,45 V, and the second part 102 has a voltage that linearly increases from 0,5 V up to 0,9 V. the first part 102 lasts for a time interval Δ1 equal to 0,40 msec, and the second part 103 lasts for a time interval Δ2 equal to 0,40 msec.

The heart stimulation device 15 guarantees a correct succession of the contractions of all the cavities of the heart that contribute to optimal pumping of the blood. Indeed, the heart stimulation device 15 provides haemodynamic advantages and low invasiveness, in particular with respect to the known stimulation devices having three electrodes.

Advantageously, the inventor has found that such specific first ventricular impulse 101 causes a pre-excitation that contributes to more quickly transmitting the second ventricular stimulus 104, thus significantly improving stroke volume and cardiac output.

The new cardiac electro-stimulation, bi-cameral with three sequential pulses, one pulse in Right Atrium, one pulse in Right Ventricle and another pulse also in Right Ventricle, has been named "FDB stimulation". FDB stimulation eliminates the negative hemodynamic effect of LBB and considerably increases both the stroke volume (SV) and the cardiac output (CO).

FDB stimulation is not only fundamental in preventing the HF in patients with PM or Def undergoing right cardiac electro-stimulation, but also has a therapeutic effect on said HF patients. FDB stimulation also is an improvement with respect to the tri-cameral stimulation CRT (Cardiac Resynchronization Therapy).

## Claims

1. Heart stimulation device (15) to stimulate the heart of a patient with a sequence of cardiac stimulation cycles, the heart stimulation device comprising
- an atrial electrode (16) adapted to be connected to the right atrium (RA) of the heart,
- a ventricular electrode (17) adapted to be connected to the right ventricle (RV) of the heart, and
- a control system programmed to emit, for each cardiac stimulation cycle:
- an atrial impulse (100) for a stimulation of the right atrium (RA) by means of the atrial electrode (16),
- a first ventricular impulse (101) in the right ventricle (RV) by means of the ventricular electrode (17), emitted after a time interval T1 from the emission of the atrial impulse (100),
- a second ventricular impulse (104) for a stimulation of the right ventricle (RV), emitted after a time interval T2 from the first ventricular impulse (101), having a voltage and a duration adapted to provoke the cardiac stimulation of the patient,
**characterized in that** the first ventricular impulse (101) is formed by
a first part (102) having a constant voltage which is not in excess of 50% of the cardiac stimulation threshold potential of the patient, and
a second part (103), subsequent to the first part (102), having an absolute value of voltage which is increasing from said constant voltage up to 70-90% of the cardiac stimulation threshold potential of the patient,
**in that** said first part (102) and said second part (103) have equal duration and **in that** said absolute value of voltage increases linearly.

2. Heart stimulation device according to claim 1, which is of the dual-chamber type for a patient having an ejection fraction less than 35% and classified in NYHA class III or IV.

3. Heart stimulation device according to anyone of the preceding claims, wherein said constant voltage is comprised between 35-50% of the cardiac stimulation threshold potential of the patient.

4. Heart stimulation device according to anyone of the preceding claims, wherein said first part (102) has a duration of about 0,4 or about 0,5 msec, preferably of 0,40 msec, and wherein said second part (103) has a duration of about 0,4 or about 0,5 msec, preferably of 0,40 msec.

5. Heart stimulation device according to anyone of the preceding claims, wherein said time interval T2 is 70 msec.

6. Heart stimulation device according to anyone of the preceding claims, wherein the cardiac stimulation threshold potential of the patient is comprised between 0,80 V and 1,40 V.

7. Heart stimulation device according to anyone of the preceding claims, wherein said constant voltage is 0,5 V, and said absolute value of voltage increases up to 1 V.

## Patentansprüche

1. Herzstimulationsvorrichtung (15) zur Stimulation des Herzens eines Patienten mit einer Sequenz von kardialen Stimulationszyklen, wobei die Herzstimulationsvorrichtung aufweist:
- eine atriale Elektrode (16), geeignet um mit dem rechten Vorhof (RA) des Herzens verbunden zu werden,
- eine ventrikuläre Elektrode (17), geeignet um mit der rechten Herzkammer (RV) des Herzens verbunden zu werden, und
- ein Steuerungssystem, das programmiert ist zum Abgeben: für jeden kardialen Stimulationszyklus
- eines atrialen Impuls (100) zur Stimulation des rechten Vorhofs (RA) mittels der atrialen Elektrode (16),
- eines ersten ventrikulären Impuls (101) in der rechten Herzkammer (RV) mittels der ventrikulären Elektrode (17), der nach einem Zeitintervall T1 nach der Abgabe des atrialen Impulses (100) abgegeben wird,
- eines zweiten ventrikulären Impuls (104) zur Stimulation der rechten Herzkammer (RV), der nach einem Zeitintervall T2 nach dem ersten ventrikulären Impuls (101) abgegeben wird und der eine Spannung und eine Dauer aufweist, die geeignet ist, die kardiale Stimulation des Patienten zu bewirken,
**dadurch gekennzeichnet, dass** der erste ventrikuläre Impuls (101) gebildet wird aus
- einem ersten Teil (102) mit einer konstanten Spannung, die nicht höher ist als 50% des kardialen Stimulationsschwellenpotentials des Patienten, und
- einem zweiten Teil (103), der auf den ersten Teil (102) folgt, mit einem Absolutwert der Spannung, die von der konstanten Spannung auf 70 - 90% des kardialen Stimulationsschwellenpotentials des Patienten ansteigt,
und dadurch, dass der erste Teil (102) und der zweite Teil (103) die gleiche Dauer aufweisen
und dadurch, dass der Absolutwert der Spannung linear ansteigt.

2. Herzstimulationsvorrichtung gemäß Anspruch 1 des Zweikammer-Typs für einen Patienten mit einer Ejektionsfraktion von weniger als 35% und der in der NYHA in Klasse III oder IV eingestuft ist.

3. Herzstimulationsvorrichtung gemäß einem der vorigen Ansprüche, wobei die konstante Spannung zwischen 35 und 50% des kardialen Stimulationsschwellenpotentials des Patienten liegt.

4. Herzstimulationsvorrichtung gemäß einem der vorigen Ansprüche, wobei der erste Teil (102) eine Dauer von etwa 0,4 oder etwa 0,5 ms, vorzugsweise 0,40 ms aufweist und wobei der zweite Teil (103) eine Dauer von etwa 0,4 oder etwa 0,5 ms, vorzugsweise 0,40 ms aufweist.

5. Herzstimulationsvorrichtung gemäß einem der vorigen Ansprüche, wobei das Zeitintervall T2 70 ms beträgt.

6. Herzstimulationsvorrichtung gemäß einem der vorigen Ansprüche, wobei das kardiale Stimulationsschwellenpotential des Patienten zwischen 0,80 V und 1,40 V liegt.

7. Herzstimulationsvorrichtung gemäß einem der vorigen Ansprüche, wobei die konstante Spannung 0,5 V beträgt und der Absolutwert der Spannung bis auf 1 V ansteigt.

## Revendications

1. Dispositif de stimulation cardiaque (15) pour stimuler le coeur d'un patient avec une séquence de cycles de stimulation cardiaque, le dispositif de stimulation cardiaque comprenant
- une électrode auriculaire (16) adaptée pour être reliée à l'oreillette droite (RA) du coeur,
- une électrode ventriculaire (17) adaptée pour être reliée au ventricule droit (RV) du coeur, et
- un système de commande programmé pour émettre, pour chaque cycle de stimulation cardiaque :
- une impulsion auriculaire (100) pour une stimulation de l'oreillette droite (RA) au moyen de l'électrode auriculaire (16),
- une première impulsion ventriculaire (101) dans le ventricule droit (RV) au moyen de l'électrode ventriculaire (17), émise après un intervalle de temps T1 à partir de l'émission de l'impulsion auriculaire (100),
- une seconde impulsion ventriculaire (104) pour une stimulation du ventricule droit (RV), émise après un intervalle de temps T2 à partir de la première impulsion ventriculaire (101), ayant une tension et une durée adaptées pour provoquer la stimulation cardiaque du patient,
**caractérisé en ce que** la première impulsion ventriculaire (101) est formée par
une première partie (102) ayant une tension constante qui ne dépasse pas 50 % du potentiel de seuil de stimulation cardiaque du patient, et
une seconde partie (103), postérieure à la première partie (102), ayant une valeur absolue de tension qui augmente à partir de ladite tension constante jusqu'à 70-90 % du potentiel de seuil de stimulation cardiaque du patient,
**en ce que** ladite première partie (102) et ladite seconde partie (103) ont une durée égale et **en ce que** ladite valeur absolue de tension augmente linéairement.

2. Dispositif de stimulation cardiaque selon la revendication 1, qui est du type à double chambre pour un patient ayant une fraction d'éjection inférieure à 35 % et classé dans la classe NYHA III ou IV.

3. Dispositif de stimulation cardiaque selon l'une quelconque des revendications précédentes, dans lequel ladite tension constante est comprise entre 35 et 50 % du potentiel de seuil de stimulation cardiaque du patient.

4. Dispositif de stimulation cardiaque selon l'une quelconque des revendications précédentes, dans lequel ladite première partie (102) a une durée d'environ 0,4 ou d'environ 0,5 ms, de préférence de 0,40 ms, et dans lequel ladite seconde partie (103) a une durée d'environ 0,4 ou d'environ 0,5 ms, de préférence de 0,40 ms.

5. Dispositif de stimulation cardiaque selon l'une quelconque des revendications précédentes, dans lequel ledit intervalle de temps T2 est de 70 ms.

6. Dispositif de stimulation cardiaque selon l'une quelconque des revendications précédentes, dans lequel le potentiel de seuil de stimulation cardiaque du patient est compris entre 0,80 V et 1,40 V.

7. Dispositif de stimulation cardiaque selon l'une quelconque des revendications précédentes, dans lequel ladite tension constante est de 0,5 V, et ladite valeur absolue de tension augmente jusqu'à 1 V.
